# EUROPEAN PATENT SPECIFICATION

(11) **EP 4 099 980 B1**
(45) Date of publication and mention of the grant of the patent: **29.11.2023**
(21) Application number: 21706998.8
(22) Date of filing: 02.02.2021
(51) Int. Cl.: A61K 9/08, A61K 31/4045, A61P 1/00

(54) **STABLE PHARMACEUTICAL COMPOSITIONS OF ROPINIROLE**
STABILE PHARMAZEUTISCHE ROPINIROLZUSAMMENSETZUNGEN
COMPOSITIONS PHARMACEUTIQUES STABLES DE ROPINIROLE

(30) Priority: 03.02.2020 FI 20205109
(43) Date of publication of application: 14.12.2022
(73) Proprietor: Orion Corporation, 02200 Espoo (FI)
(72) Inventor: LESKINEN, Mikko, 00200 Helsinki (FI); SALMIA, Jukka, 02101 Espoo (FI)
(74) Representative: J A Kemp LLP
(86) International application number: PCT/FI2021/050069
(87) International publication number: WO 2021/156540

(56) References cited:
- WO-A1-2015/044504
- US-A1- 2010 249 433

## Description

### Technical field

The present invention relates to pharmaceutical aqueous compositions, particularly solutions, containing ropinirole or a pharmaceutically acceptable salt thereof as an active ingredient. The compositions show improved chemical stability against degradation of the active ingredient. The compositions can be used, for example, in the preparation of eye drops in the field of veterinary medicine for inducing emesis in situations involving ingestion of a potentially toxic substance or foreign body by a companion animal such as a dog or a cat.

### Background of the invention

A method for inducing emesis in animals by administering an effective amount of an eye drop composition comprising a selective D₂ family dopamine agonist, such as ropinirole, as an active ingredient has been described in WO 2015/044504. An aqueous eye drop solution comprising 10 or 40 mg/ml of ropinirole hydrochloride produced vomiting rapidly and consistently in dogs without inducing more prolonged vomiting than necessary.

The development of aqueous ropinirole solutions described in WO 2015/044504 has revealed chemical instability of ropinirole in aqueous solutions. Chemical instability is considered a drawback as it is associated with a limited shelf life of the pharmaceutical product.

### Summary of the invention

It has been found that trace amounts of iron present in the composition are associated with chemical instability of ropinirole. The source of such trace amounts of iron has been found to be the impurities in the raw materials used in the preparation of the composition, particularly the impurities in the active ingredient itself, as well as the material of the vessels used in the preparation of the compositions.

Thus, according to one embodiment of the invention, the present invention provides a stable aqueous pharmaceutical composition comprising ropinirole or a pharmaceutically acceptable salt thereof in an amount of from 1 to 15 % per weight of the solution, and wherein the amount of iron is lower than 140 ppb, per weight of the composition.

According to another embodiment, the present invention provides a stable aqueous pharmaceutical composition with reduced iron content comprising ropinirole or a pharmaceutically acceptable salt thereof in an amount of from 1 to 15 %, per weight of the solution, and wherein the amount of iron is lower than 140 ppb, per weight of the composition.

According to another embodiment of the invention, the amount of iron in the above compositions is lower than 120 ppb, preferably lower than 100 ppb, more preferably lower than 80 ppb, still more preferably lower than 60 ppb, or lower than 40 ppb, per weight of the composition.

According to another embodiment, the present invention provides a method for preparing a stable aqueous pharmaceutical composition comprising ropinirole or a pharmaceutically acceptable salt thereof in an amount of from 1 to 15 % per weight of the composition, comprising the steps of
a) dissolving ropinirole or a pharmaceutically acceptable salt thereof, and optionally one or more excipients, in water; and
b) treating the mixture of step (a) with an iron scavenger.

According to another embodiment of the invention, the amount of iron after step (b) in the above method is lower than 140 ppb, preferably lower than 120 ppb, more preferably lower than 100 ppb, still more preferably lower than 80 ppb, still more preferably lower than 60 ppb, or lower than 40 ppb, per weight of the composition.

### Detailed description of the invention

The term "ppb" as used herein, refers to parts per billion per weight. Thus, 1 ppb per weight corresponds to 1 µg/kg.

The term "iron scavenger" as used herein, refers to any material that reacts with, traps, or otherwise removes iron from a solution. Examples of metal scavengers include, but are not limited to, functionalized silica, functionalized resins and activated charcoal.

The term "functionalized silica", as used herein, refers to silica having a chemical group bound thereto which acts as an iron scavenger. Examples of functionalized silica include triaminetetra-acetic acid bound to silica or sodium salt of triamine tetra-acetate bound to silica.

The term "silica", as used herein, refers to silicon dioxide.

The term "functionalized resin", as used herein, refers to polymeric material having a chemical group bound thereto acting as an iron scavenger. Examples include trimercaptotriazine bound to macroporous polystyrene.

The term "iron", as used herein, refers to chemical element with symbol Fe in solubilized form, for example in ionized or complexed form.

The term "eye drop composition", as used herein, refers to a liquid or semisolid pharmaceutical composition adapted to administration to the eye. Typical example of an eye drop composition is an ophthalmic solution to be administered dropwise to the eye.

The term "stable aqueous pharmaceutical composition", as used herein, refers to a composition which, after storage at 25 °C/60 % RH for 36 months when filled (0.7 ml) in a 1 ml blow-fill-seal LDPE ampoule under nitrogen purging and further placed in an air-tight aluminium laminate pouch under nitrogen purging, contains degradation Impurity B (4-[2-(dipropylamino)ethyl]indoline-2,3-dione hydrochloride) less than 2 %, preferably less than 1 %, per weight of the composition.

The present invention relates to a stable aqueous pharmaceutical composition, for example an eye drop composition, comprising ropinirole or a pharmaceutically acceptable salt thereof in an amount of from 1 to 15 %, preferably from 2 to 10 %, more preferably from 3 to 8 %, per weight of the solution, wherein the amount of iron is lower than 140 ppb, per weight of the solution.

According to another embodiment of the invention, the amount of iron in the above compositions is lower than 120 ppb, preferably lower than 100 ppb, more preferably lower than 80 ppb, still more preferably lower than 60 ppb, or lower than 40 ppb, per weight of the composition.

The above compositions can be prepared by a method comprising the steps of
a) dissolving ropinirole or a pharmaceutically acceptable salt thereof, and optionally one or more excipients, in water; and
b) treating the mixture of step (a) with an iron scavenger.

According to one embodiment of the invention, the amount of iron after step (b) in the above method is lower than 140 ppb, preferably lower than 120 ppb, more preferably lower than 100 ppb, still more preferably lower than 80 ppb, still more preferably lower than 60 ppb, or lower than 40 ppb, per weight of the composition.

According to one embodiment of the invention, the amount of iron after step (a) in the above method is higher than 60 ppb, or higher than 80 ppb, or higher than 100 ppb, or higher than 120 ppb, or higher than 140 ppb, or higher than 160 ppb, or higher than 200 ppb, or higher than 300 ppb, or higher than 500 ppb.

According to one embodiment of the invention, the amount of iron after step (a) in the above method is form 60 to 800 ppb, or from 80 to 800 ppb, or from 100 to 800 ppb, or from 120 to 800 ppb, or from 140 to 800 ppb, or from 160 to 800 ppb, or from 200 to 800 ppb, or from 300 to 800 ppb, per weight of the composition.

The use of the iron scavenging process in the preparation of aqueous ropinirole compositions, such as solutions, has been found to significantly improve the chemical stability of the active ingredient in the solution as iron appears to be associated with the degradation of ropinirole.

The iron scavenger used in the method is preferably a solid-phase iron scavenger. Suitable solid-phase iron scavengers include functionalized silica, functionalized resins and activated charcoal. Functionalized silica is a preferred solid-phase iron scavenger, particularly triaminetetra-acetic acid bound to silica or sodium salt of triamine tetra-acetate bound to silica, which are available as "SiliaMetS^{®} TAAcOH" and "SiliaMetS^{®} TAAcONa" from SiliCycle Inc.

The composition according to the invention can be prepared by dissolving suitable amount of ropinirole or a pharmaceutically acceptable salt thereof, for example ropinirole hydrochloride, and optionally one or more excipients, in sterilized water. If desired, the iron content of the mixture can be measured at this stage to quantify the amount of iron scavenger needed.

The solution containing ropinirole is then mixed with suitable solid-phase iron scavenger and the mixture is stirred for a time sufficient to reduce the iron content, for example 1 - 4 hours. The required amount of iron scavenger can be typically calculated from the technical manuals of the commercial iron scavenger brands. For example, about 4 to 8 molar equivalents of SiliaMetS^{®} functionalized silica products can be used in respect to the iron concentration in the mixture. Scavenging progress can be followed by normal analytical techniques. The iron content in the solution can be analysed by known analytical methods such as Inductively Coupled Plasma Mass Spectrometry (ICP-MS).

When the iron content in the mixture is sufficiently reduced, the solid-phase iron scavenger is removed by known methods, for example by filtering. Pharmaceutical excipients needed for the final pharmaceutical product can be added to the mixture if they were not added before iron scavenging step.

It is also possible to carry out the treatment step (b) by packing a solid-phase iron scavenger in a cartridge such as to use the iron scavenger in a continuous flow mode. Such cartridges are commercially available, for example, from SiliCycle Inc.

According to one embodiment, the stable aqueous pharmaceutical composition according to the present invention comprises ropinirole or a pharmaceutically acceptable salt thereof as a sole active ingredient.

The choice of suitable excipients depend on the intended use and administration route of the pharmaceutical product. In the preparation of eye drop composition, ropinirole or a pharmaceutically acceptable salt thereof can be formulated into a dosage form adapted for administration to the eye by combining the drug substance with conventional pharmaceutical diluents and carriers commonly used in eye drop compositions. The eye drop composition useful in the method of the invention may be, for example, in a liquid or semisolid form such as in the form of a solution, emulsion or suspension. A solution is particularly preferred.

Preferably, the eye drop composition is in the form of an aqueous solution adapted for administration to the eye of the animal. The concentration of ropinirole or a pharmaceutically acceptable salt thereof, for example hydrochloride salt, in a composition, for example in an eye drop composition, is suitably within the range of from about 1 to about 15 % (w/w), more typically from about 2 to about 10 % (w/w), still more typically from 3 to 8 % (w/w), per weight of the composition.

According to one embodiment, the aqueous composition, for example an eye drop composition, comprises from about 1 to about 15 %, per weight of the composition, of ropinirole or a pharmaceutically acceptable salt thereof, and from about 85 to about 99 %, per weight of the composition, of sterile water. According to another embodiment, the composition comprises from about 2 to about 10 %, per weight of the composition, of ropinirole or a pharmaceutically acceptable salt thereof, and from about 90 to about 98 %, per weight of the composition, of sterile water. According to still another embodiment, the composition comprises from about 3 to about 8 %, per weight of the composition, of ropinirole or a pharmaceutically acceptable salt thereof, and from about 92 to about 97 %, per weight of the composition, of sterile water.

The aqueous composition of ropinirole, for an example eye drop composition, may additionally comprise a tonicity agent such as sodium chloride, pH adjusting agents or buffering agents such as sodium hydroxide, hydrochloric acid, citric acid/sodium citrate, tartaric acid, fumaric acid, antioxidants such as butylated hydroxyanisole (BHA) or butylated hydroxytoluene (BHT), chelating agents such as edetate disodium, thickening agents such as sodium carboxymethylcellulose and other ingredients commonly used in the preparation of eye drop compositions.

The pH of the aqueous composition, for example an eye drop composition, comprising ropinirole or a pharmaceutically acceptable salt thereof, for example hydrochloride salt, is suitably within the range of from about 2.5 to about 8, preferably from 3 to about 6, for example from about 3.5 to about 5.

The eye drop composition can be provided in the form of a veterinary kit that comprises the eye drop composition, a package for containing said composition, and instructions for administering said composition to the eye of an animal, particularly a companion animal such as dog, for inducing emesis. Preferably, said package is an applicator, e.g. a squeezable prefilled single-use bottle, ampoule or pipette capable of dosing fixed volumes of the composition of the invention. The squeezable bottle, ampoule or pipette is preferably prepared form polymer material, such as LDPE. Suitably, the volume of the suitable bottle, ampoule or pipette ranges from about 0.5 to 5 ml. For example, about 0.5 to about 2 ml of the eye drop composition can be filled into single use blow fill seal (BFS) LDPE ampoules having volume of 0.5 ml, 1 ml or 2 ml.

The invention is further illustrated by the following examples, which are not meant to limit the scope of the invention.

### Formulation Example 1 (eye drop).

| | |
|---|---|
| Ropinirole hydrochloride | 11.4 mg (equivalent to 10 mg of ropinirole base) |
| Hydrochloric acid | to adjust to pH 4 |
| Sodium chloride | to adjust osmolality (300-400 mosm/kg) |
| Water for injection | ad 1 ml |

### Formulation Example 2 (eye drop).

| | |
|---|---|
| Ropinirole hydrochloride | 45.6 mg (equivalent to 40 mg of ropinirole base) |
| Sodium chloride | to adjust osmolality (300-400 mosm/kg) |
| Water for injection | ad 1 ml |
| | pH 4 |

### Formulation Example 3 (eye drop).

| | |
|---|---|
| Ropinirole hydrochloride | 34.2 mg (equivalent to 30 mg of ropinirole base) |
| Citric acid monohydrate | 2.5 mg |
| Sodium citrate | 2.1 mg |
| Sodium chloride | to adjust osmolality (300-400 mosm/kg) |
| Hydrochloric acid/Sodium hydroxide | if needed to adjust to pH 4 |
| Water for injection | ad 1 ml |

### Formulation Example 4 (eye drop).

| | |
|---|---|
| Ropinirole hydrochloride | 57 mg (equivalent to 50 mg of ropinirole base) |
| Citric acid monohydrate | 2.5 mg |
| Sodium citrate | 2.1 mg |
| Sodium chloride | to adjust osmolality (300-400 mosm/kg) |
| Hydrochloric acid/Sodium hydroxide if needed to adjust to pH 4 | |
| Water for injection | ad 1 ml |

### Experiment 1. Reduction of iron level using sodium salt of triamine tetra-acetate bound to silica (SiliaMetS^{®} TAAcONa obtained from SiliCycle Inc.)

102 mg of silica bound metal scavenger (SiliaMetS^{®} Triamine tetra-acetate, sodium salt) was charged to a vial. Then 10 ml of ropinirole HCl solution in water (34.2 mg/ml) containing 86 ppb of iron (Fe) was added to the vial. The mixture was stirred for 1.5 hours. Silica was removed by filtration. Iron content was measured from the filtrate. The filtrate contained 55 ppb of iron (Fe).

### Experiment 2. Reduction of iron level using triaminetetra-acetic acid bound to silica (SiliaMetS^{®} TAAcOH obtained from SiliCycle Inc.)

101 mg of silica bound metal scavenger (SiliaMetS^{®} Triamine tetra-acetic acid) was charged to a vial. Then 10 ml of ropinirole HCl solution in water (34.2 mg/ml) containing 86 ppb of iron (Fe) was added to the vial. The mixture was stirred for 1.5 hours. Silica was removed by filtration. Iron content was measured from the filtrate. The filtrate contained 40 ppb of iron (Fe).

### Experiment 3. Reduction of iron level using activated carbon (Norit SX Ultra^{®} obtained from Sigma-Aldrich Inc.)

105 mg of activated carbon (Norit SX Ultra^{®}) was charged to a vial. Then 10 ml of ropinirole HCl solution in water (34.2 mg/ml) containing 86 ppb of iron (Fe) was added to the vial. The mixture was stirred for 1.5 hours. Activated carbon was removed by filtration. Iron content was measured from the filtrate. The filtrate contained 52 ppb of iron (Fe).

### Experiment 4. Reduction of iron level using sodium salt of triamine tetra-acetate bound to silica (SiliaMetS^{®} TAAcONa obtained from SiliCycle Inc.)

101 mg of silica bound metal scavenger (SiliaMetS^{®} Triamine tetra-acetate, sodium salt) was charged to a vial. Then 10 ml of ropinirole HCl solution in water (34.2 mg/ml) containing 330 ppb of iron (Fe) was added to the vial. The mixture was stirred for 2 hours. Silica was removed by filtration. Iron content was measured from the filtrate. The filtrate contained 139 ppb of iron (Fe).

### Experiment 5. Reduction of iron level using triaminetetra-acetic acid bound to silica (SiliaMetS^{®} TAAcOH obtained from SiliCycle Inc.)

99 mg of silica bound metal scavenger (SiliaMetS^{®} Triamine tetra-acetic acid) was charged to a vial. Then 10 ml of ropinirole HCl solution in water (34.2 mg/ml) containing 330 ppb of iron (Fe) was added to the vial. The mixture was stirred for 2 hours. Silica was removed by filtration. Iron content was measured from the filtrate. The filtrate contained 81 ppb of iron (Fe).

### Experiment 6. Reduction of iron level using activated carbon (Norit SX Ultra^{®} obtained from Sigma-Aldrich Inc.)

103 mg of activated carbon (Norit SX Ultra^{®}) was charged to a vial. Then 10 ml of ropinirole HCl solution in water (34.2 mg/ml) containing 330 ppb of iron (Fe) was added to the vial. The mixture was stirred for 2 hours. Activated carbon was removed by filtration. Iron content was measured from the filtrate. The filtrate contained 122 ppb of iron (Fe).

### Experiment 7. Reduction of iron level using triaminetetra-acetic acid bound to silica (SiliaMetS^{®} TAAcOH obtained from SiliCycle Inc.)

100 mg of silica bound metal scavenger (SiliaMetS^{®} Triamine tetra-acetic acid) was charged to a vial. Then 10 ml of ropinirole HCl solution in water (34.2 mg/ml) containing 580 ppb of iron (Fe) was added to the vial. The mixture was stirred for 2 hours. Silica was removed by filtration. Iron content was measured from the filtrate. The filtrate contained 95 ppb of iron (Fe).

### Experiment 8. Stability of aqueous ropinirole HCl solution (34.2 mg/ml)

The stability of aqueous ropinirole HCl eye drop solution (34.2 mg/ml) having various iron contents were studied. The tested solution (0.7 ml) was filled in 1 ml blow-fill- seal LDPE ampoule under nitrogen purging. The sealed ampoule was placed in an air-tight aluminium laminate pouch under nitrogen purging and the pouch was maintained in 25 °C/60 % RH for 36 months. The degradation rate of ropinirole HCl was determined by measuring the degradation impurity (Impurity B, (4-[2-(dipropylamino)ethyl]indoline-2,3-dione hydrochloride) of ropinirole at release, and after 1 month, 2 months, 3 months, 24 months and 36 months. The results are shown in Table 1. It can be seen that the iron content in the solution corresponds with the degradation rate of ropinirole.

**Table 1. Chemical stability of ropinirole HCl**

| Batch | Fe / ppb | Impurity B (%), 0 mo | Impurity B (%), 1 mo | Impurity B (%), 2 mo | Impurity B (%), 3 mo | Impurity B (%), 24 mo | Impurity B (%), 36 mo |
|---|---|---|---|---|---|---|---|
| A | 184 | 0.090 | 0.173 | 0.223 | 0.313 | 0.960 | 1.846 |
| B | 137 | 0.051 | 0.082 | na | 0.128 | 0.618 | na |
| C | 87 | 0.063 | 0.087 | 0.104 | 0.119 | 0.447 | 0.595 |
| D | 66 | 0.084 | 0.110 | 0.122 | 0.129 | 0.451 | 0.712 |
| E | 47 | 0.055 | 0.079 | 0.094 | 0.130 | 0.420 | 0.587 |

| | | | | | | | |
|---|---|---|---|---|---|---|---|
| na = not analyzed Batch A does not represent the claimed invention. | | | | | | | |

### Experiment 9. Stability of aqueous ropinirole HCl solution (34.2 mg/ml)

The stability of aqueous ropinirole HCl eye drop solution (34.2 mg/ml) having iron content of 127 ppb or 37 ppb were studied. The tested solution was filled in 1 ml blow-fill- seal LDPE ampoule under nitrogen purging. The sealed ampoule was placed in an air-tight aluminum laminate pouch under nitrogen purging and the pouch was maintained in 25 °C/60 % RH for 12 months. The degradation rate of ropinirole HCl was determined by measuring the degradation impurity (Impurity B, (4-[2-(dipropylamino)ethyl]indoline-2,3-dione hydrochloride) of ropinirole at release, and after various time points. The results are shown in Table 2. It can be seen that the iron content in the solution corresponds with the degradation rate of ropinirole.

**Table 2. Chemical stability of ropinirole HCl**

| | Batch F | Batch G |
|---|---|---|
| | Iron content 127 ppb | Iron content 37 ppb |
| Time (months) | Impurity B (%) | Impurity B (%) |
| 0 | 0.118 | 0.065 |
| 1 | 0.162 | na |
| 2 | 0.215 | na |
| 3 | 0.239 | 0.142 |
| 4 | 0.273 | na |
| 5 | 0.302 | na |
| 6 | 0.354 | 0.213 |
| 9 | na | 0.300 |
| 12 | 0.506 | 0.375 |

| | | |
|---|---|---|
| na = not analyzed | | |

## Claims

1. A stable aqueous pharmaceutical composition comprising ropinirole or a pharmaceutically acceptable salt thereof in an amount of from 1 to 15 % per weight of the composition, and wherein the amount of iron is lower than 140 ppb, per weight of the composition.

2. A composition according to claim 1, wherein the amount of iron is lower than 120 ppb, per weight of the composition.

3. A composition according to claim 2, wherein the amount of iron is lower than 100 ppb, per weight of the composition.

4. A composition according to claim 3, wherein the amount of iron is lower than 80 ppb, per weight of the composition.

5. A composition according to claim 4, wherein the amount of iron is lower than 60 ppb, per weight of the composition.

6. A composition according to any of claims 1 to 5, comprising ropinirole or a pharmaceutically acceptable salt thereof in an amount of from 2 to 10 %, or from 3 to 8 %, per weight of the composition.

7. A composition according to any of claims 1 to 6, comprising 1 to 15 %, per weight of the composition, of ropinirole or a pharmaceutically acceptable salt thereof and 85 - 99 %, per weight of the composition, of sterile water; or 2 to 10 %, per weight of the composition, of ropinirole or a pharmaceutically acceptable salt thereof and 90 - 98 %, per weight of the composition, of sterile water; or 3 to 8 %, per weight of the composition, of ropinirole or a pharmaceutically acceptable salt thereof and 92 - 97 %, per weight of the composition, of sterile water.

8. A composition according to any of claims 1 to 7, which is in the form of a solution.

9. A composition according to any of claims 1 to 8, which is an eye drop composition adapted for administration to the eye of an animal, particularly a companion animal, such a dog or a cat, for inducing emesis.

10. A composition according to any of claims 1 to 9, having reduced iron content.

11. A method for preparing a stable aqueous pharmaceutical composition comprising ropinirole or a pharmaceutically acceptable salt thereof in an amount of from 1 to 15 % per weight of the composition, comprising the steps of
a) dissolving ropinirole or a pharmaceutically acceptable salt thereof, and optionally one or more excipients, in water; and
b) treating the mixture of step (a) with an iron scavenger.

12. A method according to claim 11, wherein the iron scavenger is a solid-phase iron scavenger.

13. A method according to claim 12, wherein step (b) comprises flowing the mixture of step (a) through a solid-phase iron scavenger cartridge.

14. A method according to claim 12, wherein step (b) comprises mixing the mixture of step (a) with a solid state iron scavenger followed by removing the solid state iron scavenger from the mixture.

15. A method according to any of claims 11 to 14, wherein the amount of iron after step (b) is lower than 140 ppb, or lower than 120 ppb, or lower than 100 ppb, or lower than 80 ppb, or lower than 60 ppb, per weight of the composition.

## Patentansprüche

1. Stabile wässrige pharmazeutische Zusammensetzung, umfassend Ropinirol oder ein pharmazeutisch annehmbares Salz davon in einer Menge von 1 bis 15 %, bezogen auf das Gewicht der Zusammensetzung, wobei die Menge an Eisen weniger als 140 ppb, bezogen auf das Gewicht der Zusammensetzung, beträgt.

2. Zusammensetzung nach Anspruch 1, wobei die Menge an Eisen weniger als 120 ppb, bezogen auf das Gewicht der Zusammensetzung, beträgt.

3. Zusammensetzung nach Anspruch 2, wobei die Menge an Eisen weniger als 100 ppb, bezogen auf das Gewicht der Zusammensetzung, beträgt.

4. Zusammensetzung nach Anspruch 3, wobei die Menge an Eisen weniger als 80 ppb, bezogen auf das Gewicht der Zusammensetzung, beträgt.

5. Zusammensetzung nach Anspruch 4, wobei die Menge an Eisen weniger als 60 ppb, bezogen auf das Gewicht der Zusammensetzung, beträgt.

6. Zusammensetzung nach einem der Ansprüche 1 bis 5, umfassend Ropinirol oder ein pharmazeutisch annehmbares Salz davon in einer Menge von 2 bis 10 % oder von 3 bis 8 %, bezogen auf das Gewicht der Zusammensetzung.

7. Zusammensetzung nach einem der Ansprüche 1 bis 6, umfassend 1 bis 15 %, bezogen auf das Gewicht der Zusammensetzung, Ropinirol oder ein pharmazeutisch annehmbares Salz davon und 85 - 99 %, bezogen auf das Gewicht der Zusammensetzung, steriles Wasser; oder 2 bis 10 %, bezogen auf das Gewicht der Zusammensetzung, Ropinirol oder ein pharmazeutisch annehmbares Salz davon und 90 - 98 %, bezogen auf das Gewicht der Zusammensetzung, steriles Wasser; oder 3 bis 8 %, bezogen auf das Gewicht der Zusammensetzung, Ropinirol oder ein pharmazeutisch annehmbares Salz davon und 92 - 97 %, bezogen auf das Gewicht der Zusammensetzung, steriles Wasser.

8. Zusammensetzung nach einem der Ansprüche 1 bis 7, die in Form einer Lösung vorliegt.

9. Zusammensetzung nach einem der Ansprüche 1 bis 8, bei der es sich um eine Augentropfen-Zusammensetzung handelt, die zur Verabreichung in das Auge eines Tieres, insbesondere eines Haustieres, wie eines Hundes oder einer Katze, ausgelegt ist, um Erbrechen auszulösen.

10. Zusammensetzung nach einem der Ansprüche 1 bis 9, die einen reduzierten Eisengehalt aufweist.

11. Verfahren zur Herstellung einer stabilen wässrigen pharmazeutischen Zusammensetzung, die Ropinirol oder ein pharmazeutisch annehmbares Salz davon in einer Menge von 1 bis 15 %, bezogen auf das Gewicht der Zusammensetzung, umfasst und folgende Schritte umfasst:
a) Auflösen von Ropinirol oder eines pharmazeutisch annehmbaren Salzes davon und optional eines oder mehrerer Hilfsstoffe in Wasser; und
b) Behandeln des Gemisches aus Schritt (a) mit einem Eisenfänger.

12. Verfahren nach Anspruch 11, wobei der Eisenfänger ein Festphasen-Eisenfänger ist.

13. Verfahren nach Anspruch 12, wobei Schritt (b) das Durchströmen des Gemisches aus Schritt (a) durch eine Festphasen-Eisenfängerkartusche umfasst.

14. Verfahren nach Anspruch 12, wobei Schritt (b) das Mischen des Gemisches von Schritt (a) mit einem Festphasen-Eisenfänger und das anschließende Entfernen des Festphasen-Eisenfängers aus dem Gemisch umfasst.

15. Verfahren nach einem der Ansprüche 11 bis 14, wobei die Eisenmenge nach Schritt (b) weniger als 140 ppb, oder weniger als 120 ppb, oder weniger als 100 ppb, oder weniger als 80 ppb, oder weniger als 60 ppb, bezogen auf das Gewicht der Zusammensetzung, beträgt.

## Revendications

1. Composition pharmaceutique aqueuse stable comprenant du ropinirole ou un sel pharmaceutiquement acceptable de celui-ci en une quantité de 1 à 15 % par poids de la composition, et dans laquelle la quantité de fer est inférieure à 140 ppb par poids de la composition.

2. Composition selon la revendication 1, dans laquelle la quantité de fer est inférieure à 120 ppb, par poids de la composition.

3. Composition selon la revendication 2, dans laquelle la quantité de fer est inférieure à 100 ppb, par poids de la composition.

4. Composition selon la revendication 3, dans laquelle la quantité de fer est inférieure à 80 ppb, par poids de la composition.

5. Composition selon la revendication 4, dans laquelle la quantité de fer est inférieure à 60 ppb, par poids de la composition.

6. Composition selon l'une quelconque des revendications 1 à 5, comprenant du ropinirole ou un sel pharmaceutiquement acceptable de celui-ci en une quantité de 2 à 10 %, ou de 3 à 8 %, par poids de la composition.

7. Composition selon l'une quelconque des revendications 1 à 6, comprenant 1 à 15 %, en poids de la composition, de ropinirole ou un sel pharmaceutiquement acceptable de celui-ci et 85 à 99 %, en poids de la composition, d'eau stérile ; ou 2 à 10 %, par poids de la composition, de ropinirole ou d'un sel pharmaceutiquement acceptable de celui-ci et 90 à 98 %, par poids de la composition, d'eau stérile ; ou 3 à 8 %, par poids de la composition, de ropinirole ou d'un sel pharmaceutiquement acceptable de celui-ci et 92 à 97 %, par poids de la composition, d'eau stérile.

8. Composition selon l'une quelconque des revendications 1 à 7, qui se présente sous la forme d'une solution.

9. Composition selon l'une quelconque des revendications 1 à 8, qui est une composition de collyre adaptée pour être administrée dans l'oeil d'un animal, en particulier d'un animal de compagnie, tel qu'un chien ou un chat, pour provoquer des vomissements.

10. Composition selon l'une quelconque des revendications 1 à 9, ayant une teneur réduite en fer.

11. Procédé de préparation d'une composition pharmaceutique aqueuse stable comprenant du ropinirole ou un sel pharmaceutiquement acceptable de celui-ci en une quantité de 1 à 15 % en poids de la composition, comprenant les étapes consistant à :
a) dissoudre le ropinirole ou un sel pharmaceutiquement acceptable de celui-ci, et éventuellement un ou plusieurs excipients, dans l'eau ; et
b) traiter le mélange de l'étape (a) avec un piégeur de fer.

12. Procédé selon la revendication 11, dans lequel l'éliminateur de fer est un piégeur de fer en phase solide.

13. Procédé selon la revendication 12, dans lequel l'étape (b) comprend l'écoulement du mélange de l'étape (a) à travers une cartouche piégeuse de fer en phase solide.

14. Procédé selon la revendication 12, dans lequel l'étape (b) comprend le mélange du mélange de l'étape (a) avec un piégeur de fer à l'état solide, suivi de l'élimination du piégeur de fer à l'état solide du mélange.

15. Procédé selon l'une quelconque des revendications 11 à 14, dans lequel la quantité de fer après l'étape (b) est inférieure à 140 ppb, ou inférieure à 120 ppb, ou inférieure à 100 ppb, ou inférieure à 80 ppb, ou inférieure à 60 ppb, par poids de la composition.
